# EUROPEAN PATENT APPLICATION

(11) **EP 0 819 411 A2**
(43) Date of publication of application: **21.01.1998**
(21) Application number: 97301568.8
(22) Date of filing: 10.03.1997
(51) Int. Cl.: A61F 2/02

(54) **Self-expanding stent delivery system**

(30) Priority: 15.07.1996 US 680429
(71) Applicant: ADVANCED CARDIOVASCULAR SYSTEMS, INC., Santa Clara California 95052 (US)
(72) Inventor: Limon, Timothy A., Cupertino, California 95014 (US); Saunders, Richard J., Redwood City, California 94065 (US)
(74) Representative: McLeish, Nicholas Alistair Maxwell

(57) **Abstract**

A stent-delivery catheter system delivers and implants a self-expanding stent intraluminally into the body lumen of a human patient. A self-expanding stent is removabaly attached to the distal end of an inner member so that attachment projections prevent axial movement of the stent on the inner member while the stent is being delivered and implanted in the body lumen.

## Description

The invention relates to self-expanding stent delivery systems which are used to implant a stent into a body lumen of a patient to maintain the patency of the lumen. The stent delivery system is useful in the treatment and repair of body lumens that are damaged or affected by disease, including coronary arteries, renal arteries, carotid arteries, and other body lumens.

Stents are generally cylindrically-shaped devices which function to hold open and sometimes to expand a segment of a blood vessel or other body lumen. They particularly are suitable for use to support and hold back a dissected arterial lining which can occlude the fluid passageway therethrough. Stents also are useful in maintaining the patency of a body lumen, such as a coronary artery, after a percutaneous transluminal coronary angioplasty (PTCA) procedure or an atherectomy procedure to open a stenosed area of the artery.

A variety of devices are known in the art for use as stents and include coiled wires in a variety of patterns that are expanded after being placed intraluminally by a balloon catheter; helically wound coil springs manufactured from an expandable heatsensitive material such as nickel-titanium; and self-expanding stents that are introduced into the body lumen in a compressed state and shaped in a zig-zag pattern.

Commonly, the prior art stents are delivered intraluminally through a percutaneous incision made in a femoral or renal artery. A stent is mounted on the distal end of an elongated catheter, typically on the balloon portion of a catheter, and the catheter and stent are advanced intraluminally to the site where the stent is to be implanted. With expandable stents, the balloon portion of the catheter is inflated to expand the stent radially outwardly into contact with the arterial wall, whereupon the stent undergoes plastic deformation and remains in an expanded state to hold open and support the artery.

With respect to self-expanding stents, it has been known to provide a retractable sheath, which is positioned over the self-expanding stent mounted on the distal end of a catheter. When the catheter has been advanced intraluminally to the site where the stent is to be implanted, the sheath is withdrawn, allowing the self-expanding stent to expand radially outwardly into contact with the arterial wall, thereupon holding open and supporting the artery.

One of the problems associated with the prior art stents and catheter-delivery systems is encountered in the means by which the stent is removably attached to the distal end or balloon portion of a catheter. Frequently, the means employed are insufficient to prevent the stent from being dislodged or from moving axially along the catheter or the balloon, movement which compromises the ability of the clinician to accurately and reliably deploy the stent at the desired location in the body lumen.

What has been needed and heretofore unavailable is a reliable catheter-delivery system on which the stent can be mounted and removably attached so that it does not move axially on the catheter either during delivery and advancement through the vascular system, or during implantation of the stent.

### SUMMARY OF THE INVENTION

Particular embodiments of the invention provide a self-expanding stent delivery system in which a self-expanding stent is removably attached to a catheter so that the stent remains in its position on the catheter until it is implanted. Unlike prior art stents, which may have a tendency to dislodge from the distal end of the catheter or to move axially on the catheter shaft when a protective sheath is withdrawn or when the catheter is advanced through a tortuous vasculature, these embodiments provide means for removably attaching the stent to the catheter so that it is prevented from moving axially on the catheter shaft.

A catheter assembly for removably attaching an intravascular stent is provided in which an elongated catheter has an inner member and an outer member, both extending along a longitudinal axis, wherein the inner member and the outer member have a coaxial configuration and are dimensioned so as to be free to move axially relative to each other. A self-expanding stent, having an open lattice structure, and being biased toward an open configuration, is mounted within the outer member. The inner member is slidably positioned within the lumen of the stent, and then the inner member is heated until it conforms and fills the open lattice structure of the stent with attachment projections.

One stent delivery system embodying the invention includes an inner member that is naturally pliable and deformable or, alternatively, that is heat-deformable and formed from a polymeric material which, when heated, will fill the open lattice structure of the stent with attachment projections. The inner member can be formed from polymeric materials selected from the group of materials consisting of polyurethanes, polyethylenes, polyethylterph-thalate, and nylons.

In another embodiment of the invention, an elastomeric sleeve is attached to the distal end of the inner member. This stent is mounted on the distal end of the outer member and is biased outwardly against the outer member. The inner member distal end and its sleeve are positioned within the stent, and the sleeve is heated until it fills and forms attachment projections in the open lattice structure of the stent.

Embodiments of the invention also relate to a method of mounting the self-expanding stent on the delivery catheter. The delivery catheter includes an outer member and an inner member which can move axially relative to each other and which has control handles for inducing such relative axial movement between the members. The self-expanding stent is positioned within the inner lumen of the outer member and the control handles are manipulated to slide the inner member distal end axially within the inner lumen of the self-expanding stent. Thereafter, heat is applied to the inner member distal end so that it conforms and fills the open lattice structure of the self-expanding stent with attachment projections, thereby removably attaching the self-expanding stent to the inner member distal end and preventing axial movement of the stent. The self-expanding stent remains biased radially outwardly and is prevented from expanding until expansion is desired by the outer member.

Embodiments of the invention further include a method of implanting a self-expanding stent using the catheter-delivery system described above. Using the catheter-delivery system, the stent is advanced through the vascular system of a patient until it is positioned at the site where the stent is to be implanted. The control handles are manipulated to move the inner member axially in a distal direction and to simultaneously move the outer member axially in a proximal direction. As the stent is exposed and no longer restrained by the outer member, the stent will deploy by self-expanding radially outwardly into contact with the body lumen. The stent will not move axially on the catheter shaft as the inner member and the outer member are moved axially relative to each other, because the stent is removably attached to the inner member by the attachment projections. After deployment, the catheter-delivery system is withdrawn from the patient, leaving the stent behind to perform its intended function of maintaining the patency of the fluid passageway in the body lumen.

One feature of preferred embodiments is to permit the physician to partially deploy the stent, and if it proves to have been improperly positioned, the outer member can be moved axially to recapture the partially deployed stent so that the stent can be repositioned in the proper location. For example, the control handles can be manipulated to move the inner member axially in the distal direction and to simultaneously move the outer member axially in a proximal direction to begin to deploy the stent. Thereafter, if it is determined that the stent is being implanted at the wrong location in an artery, the control handles can be manipulated to move the inner member axially in a proximal direction and to simultaneously move the outer member axially in a distal direction to recapture the partially deployed stent so that it can be repositioned in the proper location in the artery. The stent then is implanted as described above.

Other features and advantages of the present invention will become more apparent from the following detailed description of the invention, when taken in conjunction with the accompanying exemplary drawings.

### BRIEF DESCRIPTION OF THE DRAWINGS

FIGURES 1-4 represent elevational views of prior art stents and catheter-delivery systems in which the stents are self-expanding either because the stents are biased radially outwardly or are formed from a heat-sensitive material such as nickel-titanium.

FIG. 5 is a schematic view of the catheter-delivery system of one embodiment wherein the self-expanding stent is positioned within the inner lumen of the outer member before the stent is mounted on the inner member.

FIG. 6 is a schematic view depicting the inner member positioned within the inner lumen of the self-expanding stent, and a tapered mandrill inserted in the inner member for the purpose of applying heat to form attachment projections.

FIG. 7 is a schematic view depicting an alternative embodiment of the invention in which an elastomeric segment is positioned on the distal end of the inner member and is used to conform and fill in the open lattice structure of the self-expanding stent with attachment projections.

FIG. 8 is a schematic view of an over-the-wire catheter-delivery system in which the stent is being positioned at a narrowed portion of the vessel wall.

FIG. 9 is a schematic view depicting the over-the-wire catheter-delivery system of FIG. 8 in which the outer member is being withdrawn proximally so that the stent can self-expand radially outwardly into contact with the vessel wall.

FIG. 10 is a schematic view depicting the stent of FIGS. 8 and 9 being implanted and contacting the vessel wall.

FIG. 11 is a schematic view depicting a rapid-exchange catheter-delivery system in which the guide wire extends through a port in the side of catheter so that the catheter rapidly may be exchanged upon withdrawal from the patient.

FIG. 12 is a schematic view depicting the catheter-delivery system of FIG. 11 in which the stent self-expands as the outer member is withdrawn axially in the proximal direction.

FIG. 13 is a schematic view depicting the rapid-exchange catheter-delivery system in which the self-expanding stent has been implanted and brought into contact with the vessel wall, and the rapid-exchange catheter is ready to be withdrawn from the vascular system of the patient.

### DETAILED DESCRIPTION OF THE PREFERRED EMBODIMENTS

As shown in the drawings, which are included for purposes of illustration and not by way of limitation, a stent catheter-delivery system is provided in which a self-expanding stent is delivered intraluminally into the body lumen of a human patient, such as a coronary artery, carotid artery, one of the renal arteries, or the peripheral arteries or veins, and the like.

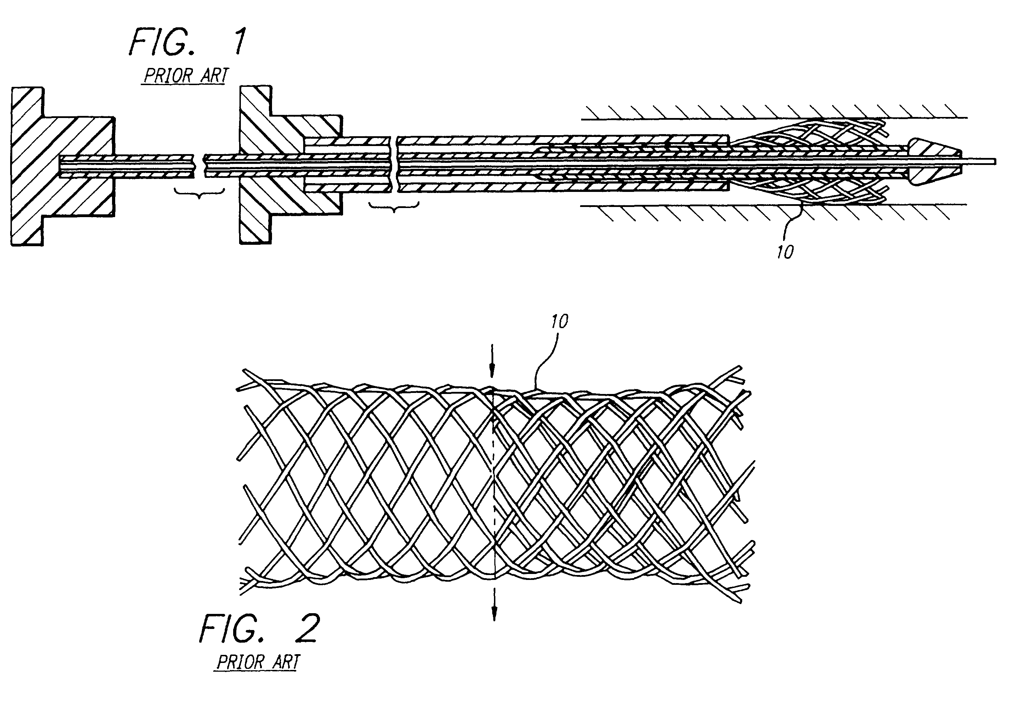

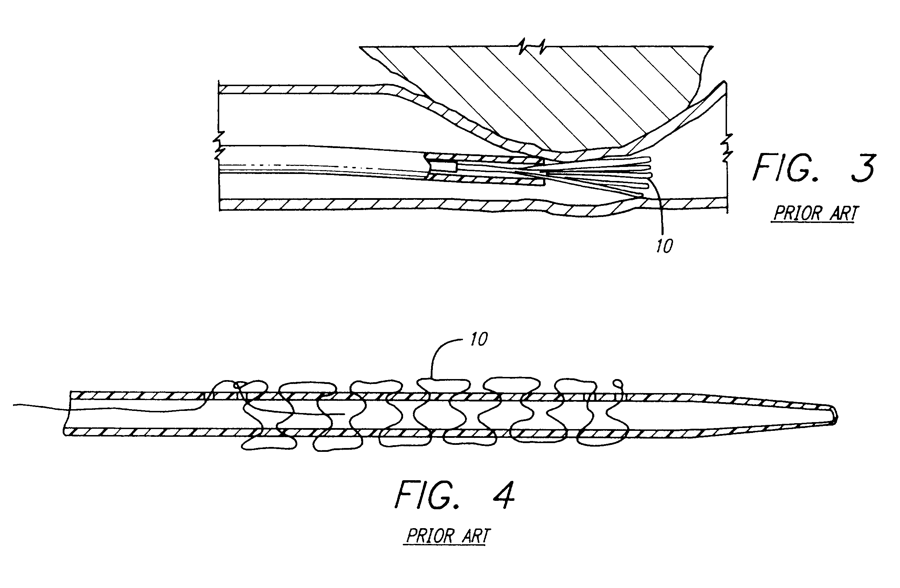

As can be seen in FIGS. 1-4, there are numerous prior art stents which are adapted for use with the illustrated stent catheter-delivery system. The stents 10 depicted in FIGS. 1-4 are all self-expanding stents and will expand from a contracted condition, i.e., the condition in which the stents are mounted on the catheter assembly, to an expanded condition in which the stent is caused to come into contact with the body lumen. The stents are self-expanding, which can be achieved by several means. As depicted in FIGS. 1-4, the prior art stents 10 are formed from a stainless steel material and are configured so as to be biased radially outwardly, intended to expand when any restraints discouraging expansion are removed. The stents depicted in FIGS. 1-4 also can be formed from a heat-sensitive material, such as nickel-titanium, which material upon application of a transformation temperature will self-expand radially outwardly. These prior art stents are representative of a large number of stents which can be adapted for use with embodiments of the invention.

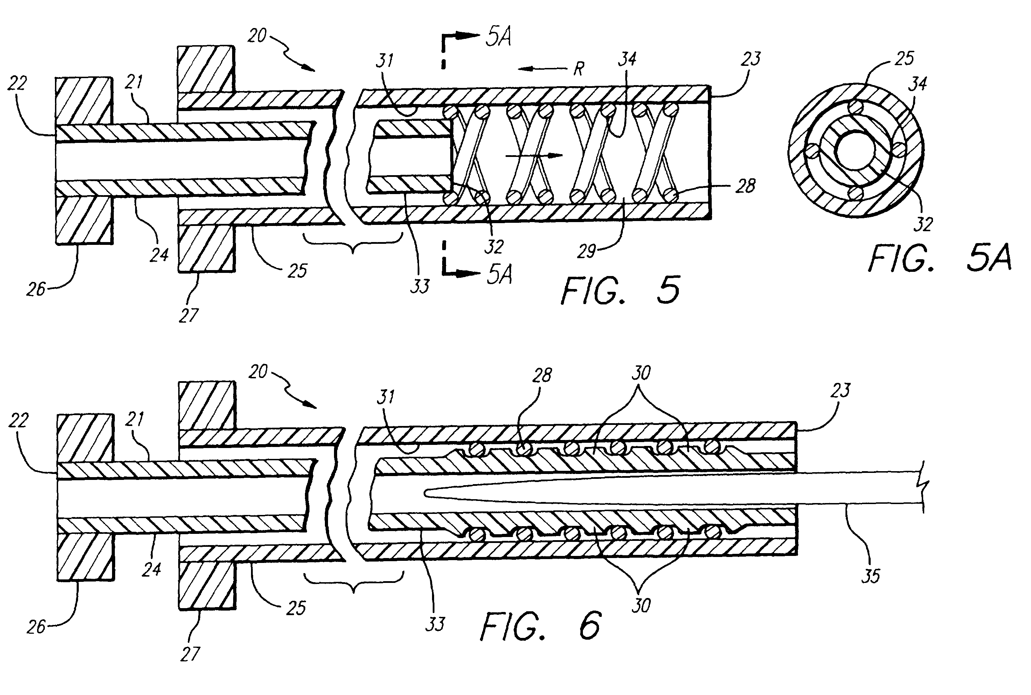

In a preferred embodiment of the invention, such as is illustrated in FIGS. 5-6, the catheter assembly 20 is provided to deliver and implant a stent. The catheter assembly 20 incorporates an elongated catheter body 21 which has a proximal end 22 and a distal end 23. The inner member 24 and an outer member 25 are arranged in coaxial alignment. The inner member 24 is slidably positioned within the outer member 25 and relative axial movement between the two members is made possible by an inner member control handle 26 and an outer member control handle 27. The control handles 26,27 can take numerous forms, but are depicted schematically for ease of illustration. As an example, however, the control handles 26,27 can take the form of a thumb-switch arrangement, a rotating-screw-type arrangement, or a ratcheting arrangement. Such control handle means are well known in prior art catheter-delivery systems.

A self-expanding stent 28 having an open lattice structure 29 is mounted on the distal end 23 of the catheter assembly 20. The self-expanding stent 28 virtually can take any configuration that has an open lattice structure 29, as can be seen in the examples of the prior art stents shown in FIGS. 1-4.

In keeping with the method of using the illustrated stent catheter-delivery system, the self-expanding stent 28 is inserted in the inner lumen 31 of the outer member 25, and positioned at the distal end 23 of the outer member. In those applications in which the self-expanding stent 28 is made from stainless steel or a similar material that is biased outwardly, the stent 28 will be compressed and inserted into the inner lumen 31 prior to delivery to the site of deployment. Thereafter, the distal end 32 of the inner member 24 is positioned within the stent inner lumen 34 so that the outer surface 33 of the inner member 24 can come into contact with the stent inner lumen 34.

In keeping with the preferred embodiment, the distal end 32 of inner member 24 is made from a polymeric material that either is soft by design, or that will become soft when heat is applied. The intent is to removably attach the self-expanding stent 28 on the outer surface 33 of inner member 24 of the inner member 24 of the elongated catheter body. The outer surface 33 of inner member 24 partially will fill the open lattice structure 29 of the stent 28 to form attachment projections 30 so that the stent cannot move in an axial direction along the outer surface 33 of the inner member 24.

In the preferred embodiment, the self-expanding stent 28 is mounted on the outer surface 33 at the distal end 32 of inner member 24 and the open lattice structure 29 is filled by attachment projections 30. Due to the coaxial arrangement between the inner member 24 and the outer member 25, the inner lumen 31 of the outer member 25 covers self-expanding stent 28 and assists in retaining the stent on the outer surface 33 of the inner member 24.

In order to conform the outer surface 33 so that it conforms or fills the open lattice structure 29 of the self-expanding stent with attachment projections 30, heat can be applied by various methods. For example, a tapered mandrel 35, as shown in FIG. 6, can be inserted in the distal end 32 of the inner member 24 in the region of the stent. Heat is then applied to the outer member 25 by means well known to those skilled in the relevant art, such as by using a heated-capture tube (not shown) surrounding the outer member 25. The capture tube can be formed from the material manufactured under the tradename "TEFLON" by the E.I. duPont de Nemours, Co., glass, or the like and generally is warmed by using heated air. As the outer member 25 is warmed, inner member 24 is inserted within the inner lumen 31 of the outer member 25, thus allowing attachment projections 30 to flow and to form around the stent 28.

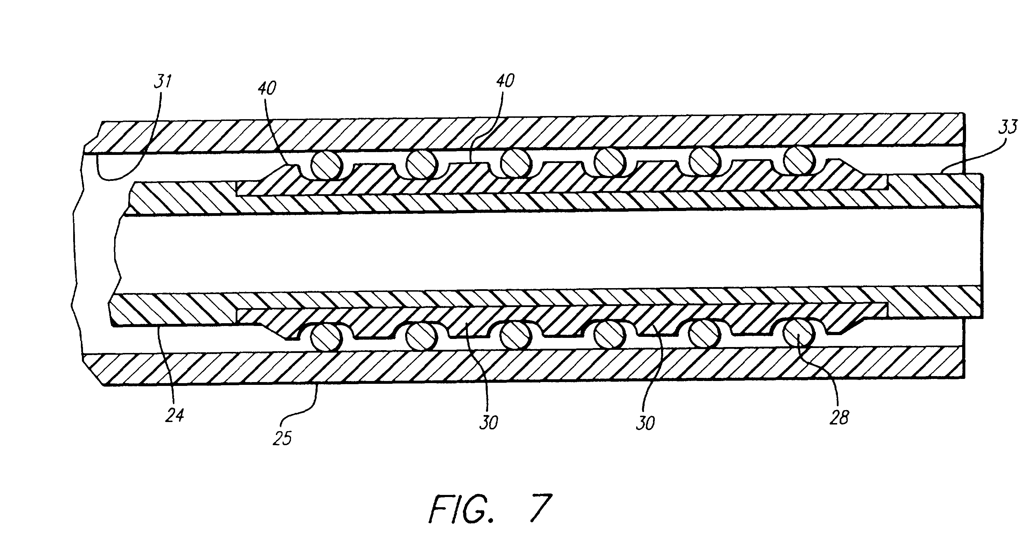

In another preferred embodiment, as depicted in FIG. 7, an elastomeric segment 40 is attached on the outer surface 33 of inner member 24 of elongated catheter body 21 at the distal end 32 of the inner member 24. The elastomeric segment 40 is formed from a heat-sensitive material, or is designed to be relatively soft as compared to the inner member 24, such that the stent 28 can be removably attached on the elastomeric segment 40, which segment will conform and fill in the open lattice structure 29 of the stent with attachment projections 30. The elastomeric segment can be heated by the aforementioned methods or, if it is formed of a material that is relatively soft, it naturally will conform and fill in the open lattice structure 29 with attachment projections 30 without the application of heat.

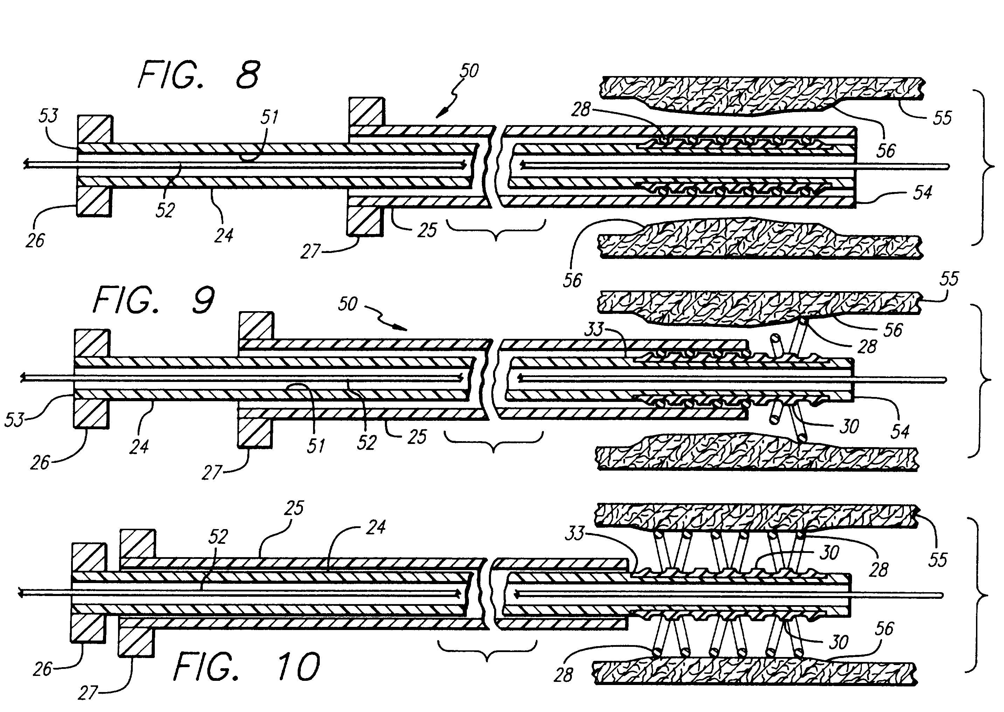

In the preferred method of use, the catheter assembly 20 is used to implant the self-expanding stent in a body lumen using an over-the-wire or rapid-exchange catheter configuration. In one preferred embodiment, as depicted in FIGS. 8-10, the over-the-wire catheter 50 has a guide wire lumen 51 which extends through the catheter and that is configured to receive the guide wire 52. In order to implant the self-expanding stent 28, the guide wire 52 is positioned in the body lumen of a patient, at a point along vessel wall 55, and typically the guide wire 52 extends past a stenosed region 56. The distal end 54 of the over-the-wire catheter 50 is threaded over the proximal end of the guide wire which is outside the patient (not shown) and the catheter 50 is advanced along the guide wire until the distal end 54 of the catheter 50 is positioned within the stenosed region 56.

As depicted in FIGS. 9 and 10, the self-expanding stent 28 is implanted in the stenosed region 56 by moving the outer member 25 of the elongated catheter body 21 in a proximal direction while simultaneously moving the inner member 24 in a distal direction. The stent 28 will not slide or move axially on the outer surface 33 because the open lattice structure 29 is filled in with attachment projections 30. As portions of the self-expanding stent 28 are no longer contained by the outer member 25, it will expand radially outwardly into contact with the vessel wall 55 in the area of the stenosed region 56. When fully deployed and implanted, as shown in FIG. 10, the stent 28 will support and will hold open the stenosed region 56 so that fluid flow is not restricted. Attachment projections 30 do not inhibit the stent 28 from self-expanding radially outwardly, but rather attachment projections 30 only impede axial movement of the stent.

With certain self-expanding stents, there is a tendency of the stent to shorten somewhat upon expansion. When stent shortening occurs, the clinician may find that the stent has been placed improperly in the stenosed region 56, if the effects of shortening previously have not been taken into consideration. Accordingly, it may be necessary, as described above, to move the inner member 24 distally in order to compensate for stent shortening upon expansion of the stent. It also is possible, due to stent design, for the self-expanding stent to not appreciably shorten upon expansion. If this is the case, it may be unnecessary to move the inner member 24 distally while simultaneously moving the outer member 25 proximally in order to release the self-expanding stent 28 in the body lumen. With a stent configuration that does not appreciably shorten during expansion, the outer member 25 is moved axially while the inner member 24 remains stationary as the self-expanding stent 28 expands radially outwardly into contact with the vessel wall 55. After the stent 28 is implanted and contacts the stenosed region 56, the over-the-wire catheter 50 is withdrawn from the vascular system of the patient. A typical over-the-wire catheter design is disclosed in U.S. Patent No. 4,323,071.

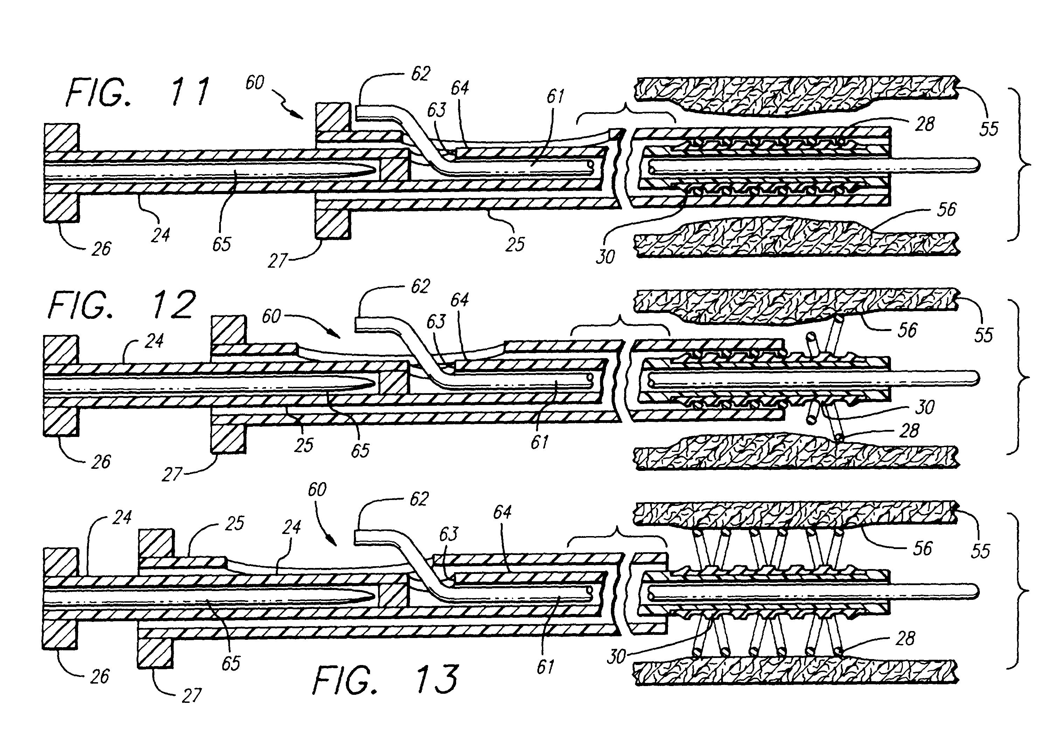

In another preferred method of implanting a stent, as is depicted in FIGS. 11-13, a rapid-exchange catheter 60 is provided. Rapid-exchange catheters are known in the art and details of the construction and use are set forth in U.S. Patent Nos. 5,458,613; 5,346,505; and 5,300,085. Generally, rapid-exchange catheters include a guide wire lumen 61 which extends in the distal portion of the catheter from a side port 63 to the distal end of the catheter. The guide wire 62 is inserted through the side port 63 and extends out of the distal end of the catheter 60 so that the distal end of the guide wire is positioned beyond the stenosed region 56. The method of deploying the self-expanding stent 28 using the rapid-exchange catheter 60 is similar to that described for using the over-the-wire catheter 50. One of the differences between the two catheter-delivery systems includes a slit 64 in the rapid-exchange catheter 60 which extends from the side port 63 to approximately just proximal of the area where the stent 28 is mounted. After the stent 28 is implanted in the stenosed region 56, the rapid-exchange catheter 60 is withdrawn from the vascular system of the patient and the guide wire 62 will peel through the slit 64, making the exchange of one catheter for another a simple process. Typically, a stiffening mandrel 65 is incorporated in the proximal region of the rapid-exchange catheter 60 to enhance the pushability of the catheter through the vascular system of the patient, and to improve the trackability of the catheter vis a vis the guide wire.

The stents as described herein can be formed from any number of materials, including metals, metal alloys and polymeric materials. Most desirably, the stents are formed from metal alloys such as stainless steel, tantalum, or the metal alloys classed as heat-sensitive such as nickel-titanium (NiTi). Stents formed from stainless steel or similar alloys typically are designed, such as in a helical coil or the like, so that the stents are spring-biased outwardly.

With respect to stents formed from shape-memory alloys such as a nickel-titanium alloy (NiTi), the stent will remain passive in its martensitic state when it is kept at a temperature below the transition temperature. In this case, the transition temperature will be below normal body temperatur, at or below 37° C (98.6° F). When the NiTi stent is exposed to normal body temperature, it immediately will attempt to return to its austenitic state, and will rapidly expand radially outwardly to achieve its preformed state. Details relating to the properties of devices made from nickel-titanium can be found in "Shape-Memory Alloys," Scientific American, Vol. 281, pages 74-82 (Nov. 1979).

With respect to all of the embodiments disclosed above, inner member 24, and for that matter outer member 25, can be formed from polymeric materials including polyurethanes, polyethylenes, polyethylterpthalate, and nylons. Similarly, the elastomeric segment 40 can be formed from polyurethane, elastomeric polyesters and the like. Generally speaking, the more proximal portions of the inner member 24 and the outer member 25 will be formed of a polymeric material that is stiffer than the distal section so that the proximal section has sufficient pushability to advance through the vascular system of the patient. On the other hand, the more distal portions of the inner member 24 and the outer member 25 can be formed of a more flexible material so that the distal portion of the catheter will remain flexible and will track more easily over the guide wire.

Other modifications and improvements may be made without departing from the scope of the invention. For example, the various drawing figures depict several configurations of the stent including various sizes, which can be modified to suit a particular application without departing from the scope of the invention. Further, the configuration of the catheter assembly is a coaxial arrangement between the inner member and the outer member, which can be modified to other configurations without departing from the invention.

## Claims

1. A catheter assembly (20) for removably attaching an intravascular stent to a delivery catheter, comprising:
an elongated catheter (21) having a proximal end (22) and a distal end (23);
the catheter having an inner member (24) and an outer member (25) extending along a longitudinal axis, the inner member and the outer member having a coaxial configuration and dimensioned for relative axial movement;
means for providing relative axial movement between the inner member and the outer member;
a self-expanding stent (28) having an open lattice structure (29), the stent being biased toward an open configuration and being positioned within a distal end (23) of the outer member (25); and
means for conforming a distal end (32) of the inner member (24) to removably attach the stent to the inner member distal end (32).

2. The catheter assembly of claim 1, wherein the means for conforming the distal end (32) of the inner member (24) to removably attach the stent, includes a plurality of attachment projections (30) formed from the inner member (24) having a heat-deformable polymeric material.

3. The catheter assembly of claim 2, wherein the polymeric material on the distal end (32) of the inner member (24) is taken from the group of polymeric materials consisting of polyurethanes, polyethylenes, polyethylterpthalate, and nylons.

4. The catheter assembly of any of the preceding claims, wherein the means for providing relative axial movement between the inner member and the outer member includes a control handle (26,27) positioned at the proximal end (22) of the elongated catheter (21).

5. The catheter assembly of any of the precending claims, wherein the self-expanding stent (28) is formed from a metal alloy taken from the group of metal alloys consisting essentially of stainless steel, nickel-titanium, and tantalum.

6. The catheter assembly of any of the preceding claims, wherein the inner member (24) of the elongated catheter (21) has a through-lumen (51) for receiving a guide wire (52) so that the elongated catheter can be positioned within a body lumen by advancing it over the guide wire.

7. The catheter assembly of any of the preceding claims, wherein the inner member (24) of the elongated catheter has a side port (62) for receiving a guide wire (52), the side port being positioned so that the catheter can be rapidly exchanged.

8. The catheter assembly of any of the preceding claims, wherein the distal end (23) of the elongated catheter includes a polymeric sleeve (40) attached to the distal end (23), the polymeric sleeve adapted to conform and fill the lattice structure of the self-expanding stent with a plurality of attachment projections (30).

9. A method of mounting an intravascular stent on a delivery catheter, the method comprising:
providing a delivery catheter having an elongated catheter body (21) and a proximal end (22) and a distal end (23), the catheter having an inner member (24) and an outer member (25) extending along a longitudinal axis, the inner member and the outer member having a coaxial configuration and dimensioned for relative axial movement, and control handles (26,27) for providing relative axial movement between the inner member and the outer member;
positioning a self-expanding stent (28) within an inner lumen (31) of the outer member (25);
manipulating the control handles to slide the inner member distal end within an inner lumen (34) of the self-expanding stent; and
heating the inner member distal end (32) so that it conforms and fills the open lattice structure of the self-expanding stent with a plurality of attachment projections (30), thereby removably attaching the self-expanding stent to the inner member distal end.

10. A method of implanting a self-expanding stent (10) in a body lumen, the method comprising:
providing an elongated catheter (21) having a proximal end (22) and a distal end, the catheter having an inner member and an outer member extending along a longitudinal axis (24), the inner member and the outer member having a coaxial configuration and dimensioned for relative axial movement, control handles (26,27) for providing relative axial movement between the inner member and the outer member and a self-expanding stent mounted on a distal end of the inner member with the distal end of the outer member forming a sheath around the self-expanding stent;
manipulating the control handles to simultaneously move the inner member axially in a distal direction and the outer member axially in a proximal direction;
deploying the stent by permitting it to self-expand radially outwardly into contact with the body lumen while preventing axial movement of the stent on the catheter by providing a plurality of attachment projections (30); and
withdrawing the catheter from the body lumen.

11. The method of implanting a stent (10) of claim 10, the method further comprising:
positioning the distal end (23) of the catheter (21) at the site for implanting the stent;
manipulating the control handles to simultaneously move the inner member axially in a distal direction and the outer member axially in a proximal direction to begin implanting the stent;
manipulating the control handles (26,27) to simultaneously move the inner member (24) axially in a proximal direction and the outer member (25) axially in a distal direction to recapture the stent and to position the stent on the inner member distal end;
repositioning the catheter distal end to implant the stent;
manipulating the control handles to simultaneously move the inner member axially in a distal direction and the outer member axially in the proximal direction to release the stent to self-expand in the body lumen; and
withdrawing the catheter from the body lumen.

12. A method of implanting a self-expanding stent (10) in a body lumen, the method comprising:
providing an elongated catheter (21) having a proximal end (22) and a distal end (23), the catheter having an inner member (24) and an outer member (25) extending along a longitudinal axis, the inner member and the outer member having a coaxial configuration and dimension for relative axial movement, control handles (26,27) for providing relative axial movement between the inner member and the outer member and a self-expanding stent mounted on a distal end of the inner member with the distal end of the outer member forming a sheath around the self-expanding stent;
manipulating the control handles to move the outer member axially in a proximal direction while maintaining the inner member stationary;
deploying the stent by permitting it to self-expand radially outwardly into contact with the body lumen; and
withdrawing the catheter from the body lumen.

13. The method of implanting a stent (10) of claim 12, the method further comprising:
positioning the distal end (23) of the catheter at the site for implanting the stent;
manipulating the control handles (26,27) to simultaneously move the inner member axially in a proximal direction and the outer member axially in a distal direction to recapture the stent;
repositioning the catheter distal end to implant the stent;
manipulating the control handles to move the outer member axially in the proximal direction to release the stent to self-expand in the body lumen while simultaneously molding the inner member stationary; and
withdrawing the catheter from the body lumen.
